# EUROPEAN PATENT APPLICATION

(11) **EP 2 124 057 A1**
(43) Date of publication of application: **25.11.2009**
(21) Application number: 07858289.7
(22) Date of filing: 05.12.2007
(51) Int. Cl.: G01N 33/543

(54) **METHOD FOR ORIENTED IMMOBILISATION OF ANTIBODIES ON SOLID MEDIA, RESULTING DEVICES AND USES THEREOF**

(30) Priority: 27.12.2006 ES 200603289
(71) Applicant: Consejo Superior De Investigaciones Científicas, 28006 Madrid (ES)
(72) Inventor: BATALLA BOSQUET, Pilar, E-28049 Cantoblanco (ES); FUENTES GARCÍA, Manuel, E-28049 Cantoblanco (ES); GRAZU BONAVIA, Valeria, E-28049 Cantoblanco (ES); MATEO GONZÁLEZ, César, E-28049 Cantoblaco (ES); FERNÁNDEZ LAFUENTE, Roberto, E-28049 Cantoblanco (ES); GUISÁN SEIJAS, Jose Manuel, E-28049 Cantoblanco (ES)
(74) Representative: Pons Ariño, Angel
(86) International application number: PCT/ES2007/070205
(87) International publication number: WO 2008/077984

(57) **Abstract**

The invention describes a method for covalent oriented immobilisation of antibodies which is achieved by a step of absorbing the antibody by anionic exchange, a step of covalent bonding the absorbed antibody and the medium, and subsequently inerting or blocking of the surface of the medium. Also included are the actual inert device serving as medium for the immobilised and oriented antibodies and produced by means of the method of the invention

## Description

### TECHNICAL FIELD OF THE INVENTION

The patent of the invention belongs to the field of antibody support devices, which would be applied in the preparation of affinity or immunosensor columns, and in the fields of biotechnology, biocatalysis, fermentation, purification of proteins or enzymes, biomedicine, the environment, preventive detection of toxins, nutrition, water monitoring and biosafety.

### STATE OF THE ART

The high specificity of antibodies has made them essential tools in immunoaffinity and the development of biosensors. However, for both applications, the antibodies must be immobilised and the immobilisation step may define the potential applications of the immobilised antibodies (see references 1-7 and 10-11).

An optimal method of immobilising antibodies for any of the potential applications should meet a number of requirements, depending on the type of system whereto it is to be applied:
1. The immobilisation should not cause great distorsions in the antibody's recognition site.
2. The recognition area must be as exposed as possible to the environment. This is essential if they are intended to be used in the recognition of biomacromolecules or cells, or if the reading is to be performed using a second antibody or biomacromolecule, since only molecules the active centre whereof is exposed to the environment will be able to adsorb the macromolecules, such that the percentage of well-oriented molecules will determine the rate of adsorption of the analyte (biosensors) or the column's loading capacity.
3. The final support surface must be as inert as possible, in order to prevent unspecific adsorptions of other molecules onto the support, which would complicate the final analyses. In bioaffinity columns, the unspecific adsorption of any other compound would drastically reduce the potential use of this type of purification technique. In the case of biosensors, an unspecific adsorption may also reduce the potential applications thereof, but special emphasis should be placed on preventing the unspecific adsorption of any of the components involved in the reading of the signal (e.g. protein-labelled antibodies).

Recently, a protocol has been disclosed that makes it possible to immobilise antibodies with these characteristics (5 and 6). The method is based on the immobilisation of the antibody through the periodate-oxidised glycosylated chains: the antibody is covalently immobilised on aminated supports, which are capable of adsorbing proteins by anion exchange, and, in order to prevent it, a blockage step is performed using aldehyde-aspartic-dextran; this step must be very strictly monitored in order to prevent the chemical modification of the antibody's recognition sites. This method makes it possible to recover antibodies with up to 60%-70% functionality without any adsorption of proteins of crude extracts of different microorganisms. However, the method is complex when scaling up to the industrial level, and complicated if the support is to be coated with antibodies, due to the relative low rate of the covalent reaction between oxidised sugar and primary amines.

### DESCRIPTION OF THE PATENT

### BRIEF DESCRIPTION

One object of this invention is a method for the oriented covalent immobilisation of antibodies, hereinafter method of the invention, which comprises the following steps:
i) antibody adsorption by anion exchange on a bifunctional solid surface or support, activated with two types of groups: ionised amino groups from the support, composed of groups capable of acting as anion exchangers to define the site of interaction between the antibody and the support, and groups capable of covalently reacting with and immobilising the antibody at low ionic strength,
ii) covalent binding of the already-adsorbed antibody and the support, the conditions whereof will depend on the activated group, and
iii) inertisation or blockage of the support surface.

Another object of the invention is the use of the method of the invention to produce a support device for oriented immobilised antibodies, wherein the support is inert.

Finally, another object of the invention is the inert support device for oriented immobilised antibodies prepared with the method of the invention.

### DETAILED DESCRIPTION

This invention is based on the fact that the inventors have observed that the oriented covalent immobilisation of an antibody may be performed on a heterofunctional aminated support (with groups that allow for the covalent immobilisation of the previously adsorbed antibody) at pH 5 to 9, primarily involving the antibody's heavy chains; this causes the recognition sites to be completely free and capable of recognising biomacromolecules, whilst preserving their biological activity; furthermore, they adsorb their corresponding antigens very rapidly and specifically.

The final blockage of the support surface with the antibody makes it inert toward the unspecific adsorption of any other type of proteins (albumin, immunoglobulins, crude extracts of Escherichia coli, etc.). This method of immobilisation may be performed on all types of pre-existing solid supports and for all types of applications of immobilised antibodies. On the one hand, the immobilisation of antibodies on agarose gels is useful in the preparation of immunoaffinity supports for the selective adsorption of enzymes and proteins. On the other hand, the immobilisation of antibodies on magnetic particles would be very useful for the detection of traces of cells, proteins and any analytes of interest present in complex mixtures (blood, foods, wastewaters, etc.).

This patent shows various strategies that make it possible to recover a high antibody functionality on a final inert support and surface, starting from different solid supports and activated groups. The common link between all of them is the method of immobilisation, which takes place in 3 steps on supports activated with two types of groups: groups capable of acting as anion exchangers to define the site of interaction between the antibody and the support, and groups capable of covalently reacting with the antibody. The three steps are:
i) antibody adsorption by anion exchange on a bifunctional or multifunctional solid surface or support activated with ionised amino groups and with functional groups capable of covalently immobilising proteins (this step takes place very rapidly) at low ionic strength,
ii) covalent binding between the already-adsorbed antibody and the support, depending on the activated group, and
iii) inertisation or blockage of the support surface (e.g. making the support surface, which is an anion exchanger, into a very hydrophilic surface with a net zero charge).

These supports are activated with two types of groups: groups capable of acting as anion exchangers to define the site of interaction between the antibody and the support, and groups capable of covalently reacting with the antibody.

Therefore, one object of this invention is a method for the oriented covalent immobilisation of antibodies, hereinafter method of the invention, which comprises the following steps:
i) antibody adsorption by anion exchange on a bifunctional solid surface or support activated with two types of groups: ionised amino groups from the support, composed of groups capable of acting as anion exchangers to define the site of interaction between the antibody and the support, and groups capable of covalently reacting with and immobilising the antibody at low ionic strength,
ii) covalent binding between the already-adsorbed antibody and the support, the conditions whereof will depend on the activated group, and
iii) inertisation or blockage of the support surface.

Another particular object of the invention is the method of the invention wherein supports are used that are activated with two types of groups: positively charged groups (for example, amino groups) and groups capable of covalently reacting with the proteins, belonging, for illustrative purposes and without this limiting the scope of the invention, to the following groups: epoxide, aldehyde or glutaraldehyde groups.

Another particular object of the invention is the method of the invention wherein the bifunctional support with positively charged groups is a support that is activated with a low density of glutaraldehyde-activated primary amino groups, capable of adsorbing the antibody by anion exchange at very low ionic strength and, subsequently, covalently immobilising the antibody; as a result, the support is not capable of adsorbing proteins at moderate ionic strength.

Another particular object of the invention is the method of the invention wherein the bifunctional support with positively charged groups is a support that is activated with a low density of epoxide groups on a space arm with an amino group; the amino groups are capable of adsorbing the antibody by anion exchange at very low ionic strength, but incapable of adsorbing other proteins at moderate ionic strength; subsequently, the epoxide groups are capable of covalently immobilising the antibody, and the remaining epoxide groups are blocked or made inert with different molecules that lead to a neutral final charge (for example, mercaptoethanol, Gly and Cys) or a negative final charge (for example, Asp and Glu), such that the final charge balance is close to 0.

Another particular object of the invention is the method of the invention wherein the bifunctional support with positively charged groups is a support that is activated with a high density of glutaraldehyde groups on amino groups; the amino groups will be capable of adsorbing the antibody by anion exchange at very low ionic strength and, subsequently, the glutaraldehyde groups are capable of covalently immobilising the antibody; in order to eliminate the support's adsorption capacity, it is coated with a layer of small molecular size proteins.

A particular embodiment of the invention is the method of the invention wherein the space arm of the support with glutaraldehyde-activated amino groups is an amino acid ester or similar; the amino groups will be capable of adsorbing the antibody by anion exchange at very low ionic strength and, subsequently, the glutaraldehyde groups will be capable of covalently immobilising the antibody; in order to eliminate the support's adsorption capacity, it is sufficient to hydrolise the ester to generate a carboxyl group that belongs, for illustrative purposes and without this limiting the scope of the invention, to the following groups: cysteine methyl ester, which is made to react with an epoxide support, an ester of an amino acid such as Lys, which is made to react on a support activated with carboxyl groups, or an ester of an amino acid such as Asp or Glu, which is made to react on a support activated with amino groups.

Another particular object of the invention is the method of the invention wherein the support is activated with a high density of epoxide groups with a space arm that contains at least one positively charged group; this positive charge is capable of promoting antibody adsorption by anion exchange at very low ionic strength, and, subsequently, immobilising the antibody by covalent reacton; in order to eliminate the support's adsorption capacity, it is coated with a layer of small molecular size proteins or the epoxide groups are blocked with molecules that have a net negative charge (e.g. Asp and Glu).

Another particular object of the invention is the method of the invention wherein the support is activated with epoxide groups, ideally one-third whereof have been modified with diamines (e.g. ethylenediamine) with two cationic groups; these amino groups will be capable of adsorbing the antibody by anion exchange at very low ionic strength and, subsequently, the remaining epoxide groups are capable of covalently immobilising the antibody; in order to eliminate the support's adsorption capacity, it is coated with a layer of small molecular size proteins or the epoxide groups are blocked with molecules that have a net negative charge (e.g. Asp and Glu).

The final blockage of the support surface with the antibody makes it inert toward the unspecific adsorption of any other type of proteins (albumin, immunoglobulins, crude extracts of Escherichia coli, etc.).

Another particular object of the invention is the method of the invention wherein the support is porous and belongs, for illustrative purposes and without this limiting the scope of the invention, to the following groups: agarose, glass, silica and epoxy-acrylic.

Another particular object of the invention is the method of the invention wherein the support is a non-porous magnetic nanoparticle.

Another particular object of the invention is the method of the invention wherein the immobilised antibody is used in affinity chromatography or as an immunosensor.

Another particular object of the invention is the method of the invention wherein the support is used as an immunosensor or in affinity chromatography in mixtures with particles in suspension; for example, membranes, spores or cells.

Another object of the invention is the use of the method of the invention to produce a support device for oriented immobilised antibodies wherein the support is inert.

This method of immobilisation may be performed on all types of pre-existing solid supports and for all types of applications of immobilised antibodies. For example, the device may consist of agarose gels with immobilised antibodies, which are useful in the preparation of immunoaffinity supports for the selective adsorption of enzymes and proteins; in magnetic particles for the detection of traces of cells, proteins and any analytes of interest present in complex mixtures (blood, foods, wastewaters, etc.).

Therefore, another object of the invention is the inert support device for oriented immobilised antibodies prepared with the method of the invention.

### EXAMPLES OF THE INVENTION

### Example 1. Antibody immobilisation on supports activated with glutaraldehyde groups.

Glutaraldehyde groups are capable of immobilising proteins in a direct, covalent manner, but the reactivity is not very high and, at neutral pH and low ionic strength, protein adsorption is much faster than covalent binding, such that the antibody will bind to the support on the area that is richest in negative charges (the heavy chains); thereafter, given the proximity of the antibody's nucleophilic groups and the support's glutaraldehyde groups, covalent immobilisation of the antibody on the support will take place (12). Subsequently, it will be necessary to reduce these supports' capacity to adsorb or covalently immobilise proteins in order for the final surface to be completely inert. There are several possibilities:

### 1.1 Antibody immobilisation on 4BCL agarose (support with a low degree of activation of amino groups) with 3 micromoles of glutaraldehyde-activated MANAE groups/g.

5 mg of anti-peroxidase antibody in 50 ml of 5 mM phosphate buffer at pH 7 were offered to 5 grams of 4BCL agarose with 3 micromoles of glutaraldehyde-activated MANAE groups (e.g. following incubation with 10% glutaraldehyde at pH 7 for 16 hours) (12). In this type of supports, the antibodies will be adsorbed by ionic exchange at very low ionic strength (10 mM of phosphate) and, subsequently, will react with the support's glutaraldehyde groups. Due to the low degree of activation, these supports will not be capable of adsorbing proteins at 100 mM of sodium phosphate (13). Therefore, directed immobilisation of the antibody is achieved in the desired area on a reasonably inert surface.

After 24 hours, 60% of the antibody had become covalently immobilised on the support, as verified by the non-desorption thereof at high ionic strength (although the first step was performed via an adsorption by ionic exchange on the support's amino groups). At this time, the derivative was reduced, in order to eliminate the reactivity of glutaraldehyde, which, albeit very slowly, could immobilise molecules contained in the samples, by raising the pH to 8.5, and the immobilised preparation was reduced with 1 mg/ml of sodium borohydride for 30 minutes.

In following this protocol, the main problem would lie in that the rate of antibody adsorption would not be very high, which might make it difficult to completely coat the support surface with antibody molecules.

Subsequently, in order to verify the inertisation of the support, 1 gram of immobilised antibody was incubated with 1 gram of proteins of a crude extract of *E. coli* in 100 ml of 100 mM of sodium phosphate at pH 7, and no adsorption of proteins was detected after 3 hours of incubation. When peroxidase was added to this crude extract, the former was very quickly adsorbed onto the immobilised antibody preparation. The functionality of the immobilised antibody was over 80% of that expected on the basis of the immobilised antibody molecules.

### 1.2. Antibody immobilisation on glutaraldehyde-activated aminated magnetic particles (100 micromoles of amino groups/g of support).

5 mg of anti-peroxidase antibody in 50 ml of 5 mM phosphate buffer at pH 7 were offered to 5 grams of glutaraldehyde-activated support (aminated magnetic particles) (e.g. following incubation with 10% glutaraldehyde at pH 7 for 16 hours) (12). After 2 hours, 100% of the antibody had become covalently immobilised on the support (although the first step was primarily performed via an adsorption by ionic exchange on the support's amino groups, which takes place very rapidly when performed at low ionic strength). The covalent bond was verified by the non-desorption of the antibody at high ionic strength.

However, these supports maintain their capacity as anion exchangers, for which reason, even after reducing the glutaraldehyde groups with borohydride, this preparation was capable of adsorbing up to 90 mg of proteins/g of support when a crude extract of *E*. *coli* was offered thereto; consequently, it was necessary to make the support surface inert.

In order to reduce said exchange capacity, the support may be blocked with small inert proteins (naturally glycosylated proteins or proteins chemically modified with dextrans), such that it may not adsorb any proteins; more specifically, the pH was raised to 9, and 1 g of a small or inert protein, such as chymotrypsinogen (subject to de-activation with an irreversible covalent inhibitor in order to eliminate the remaining protein activity), was offered to the support with the immobilised anti-peroxidase antibody. After 16 hours, to allow for the support surface to become coated with the small-size protein, the immobilised preparation was reduced by adding 1 mg/ml of sodium borohydride for 30 minutes at pH 8.5-10, in order to eliminate the glutaraldehyde groups' reactivity. Subsequently, 1 g of the immobilised antibody preparation was incubated with 1 g of proteins of a crude extract of *E*. *coli* in 100 ml of 100 mM of sodium phosphate at pH 7, and no unspecific adsorption of proteins was detected after 3 hours of incubation.

When peroxidase was added to this crude extract, this protein was very quickly adsorbed onto the immobilised antibody preparation. The antibody functionality was over 80% of that expected on the basis of the immobilised antibody molecules.

### 1.3. Antibody immobilisation on a support coated with amino acid esters with the amino group activated with glutaraldehyde.

Another option of this invention is the use of supports coated with amino acid esters with the amino group activated with glutaraldehyde.

At this point, there were several options to prepare the support, such as, for example, binding of Cys methyl ester to epoxide supports, Lys esters or similar bound to supports carboxylated by amide bonds, Asp, Glu or similar esters bound to supports aminated also by amide bonds, etc. In this manner, it will be possible to prepare supports with a high density of aminated groups that may be activated with glutaraldehyde, thereby generating anion exchanger supports similar to those in the preceding example, where adsorption of the antibody and the covalent binding thereof will be very rapid.

### 1.3.1. Antibody immobilisation on a support coated with amino acid esters (Eupergit C with 210 micromoles of Cys methyl ester groups) with the amino group activated with glutaraldehyde.

In a first specific example, the support was obtained by incubating a support with epoxide groups (in this case, Eupergit C) in 3 M Cys methyl ester at pH 6 for 30 hours. Subsequently, the support was washed and made to react with 10% glutaraldehyde at pH 7 for 24 hours. 5 mg of anti-peroxidase antibody in 50 ml of 5 mM phosphate buffer at pH 7 were offered to 5 grams of this support. After 2 hours, 100% of the antibody had become covalently immobilised on the support, verified as in the preceding cases (although the first step was performed via an adsorption by ionic exchange on the support's amino groups). At this point, the pH was raised to 8.5 (possible at pH between 8.5 and 10) and the immobilised preparation was reduced with 1 mg/ml of sodium borohydride for 30 minutes, in order to eliminate the glutaraldehyde groups' reactivity.

Even after reducing the glutaraldehyde groups with borohydride, this preparation was capable of adsorbing up to 100 mg of proteins/g of support when a crude extract of *E. coli* was offered thereto; consequently, it was necessary to make the support surface inert.

In order to achieve this objective, the immobilised antibody was incubated at pH 10 for 48 h (also at pH 4 or pH 9 for 72 hours) in order to promote hydrolysis of the methyl ester, which made it possible to create a carboxyl group that, at pH 7, exhibited a negative charge; i.e. for each amino acid molecule, one negative charge will be generated, thereby creating a highly hydrophilic support, but without a net charge in the pH range between 5 and 9. Therefore, this support will not be capable of unspecifically adsorbing proteins under a wide range of conditions.

Subsequently, 1 g of the immobilised antibody was incubated with 1 g of proteins of a crude extract of *E. coli* in 100 ml of 100 mM of sodium phosphate at pH 7, and no adsorption of proteins was detected after 3 hours of incubation. When peroxidase was added to this crude extract, this protein was very quickly adsorbed onto the immobilised antibody preparation. The immobilised antibody functionality was over 80% of that expected on the basis of the immobilised antibody molecules.

### 1.3.2. Antibody immobilisation on a 4BCL agarose support with 60 micromoles of glutaraldehyde-activated Lys methyl ester groups.

A second example of supports coated with amino acid esters was performed using glutaraldehyde-activated Lys methyl ester groups bound to supports carboxylated by amide bonds.

More specifically, the support was prepared from carboxymethyl agarose, activating the carboxyl groups with 100 mM of carbodiimide at pH 7 in the presence of 3 M of Lys methyl ester.

Subsequently, the support was made to react with 10% glutaraldehyde at pH 7 for 24 hours. 5 mg of anti-peroxidase antibody in 50 ml of 5 mM phosphate buffer at pH 7 were offered to 5 g of this support. After 2 hours, 100% of the antibody had become covalently immobilised on the support (although the first step was performed via an adsorption by ionic exchange on the support's amino groups). At this point, the pH was raised to 8.5 and the immobilised preparation was reduced with 1 mg/ml of sodium borohydride for 30 minutes, in order to eliminate the glutaraldehyde groups' reactivity.

Even after reducing the glutaraldehyde groups with borohydride, this preparation was capable of adsorbing up to 40 mg of proteins/g of support when a crude extract of *E. coli* was offered thereto; consequently, it was necessary to make the support surface inert. In order to achieve this objective, the immobilised antibody was incubated at pH 10 for 48 h in order to promote hydrolysis of the methyl ester, which made it possible to create a carboxyl group that, at pH 7, would have a negative charge.

Subsequently, 1 g of the immobilised antibody was incubated with 1 g of proteins of a crude extract of *E. coli* in 100 ml of 100 mM of sodium phosphate at pH 7, and no adsorption of proteins was detected after 3 hours of incubation. When peroxidase was added to this crude extract, this protein was very quickly adsorbed onto the immobilised antibody preparation. The immobilised antibody functionality was over 80% of that expected on the basis of the immobilised antibody molecules.

### Example 2. Amino-epoxide supports.

Epoxide groups are capable of reacting with a large number of protein residues, but in an extremely slow manner. However, if the proteins are adsorbed onto the support by any other mechanism, the proximity between the epoxide groups and the support's nucleophilic groups will allow for covalent binding between the support and the antibody (8). Thus, if the first step is via cation exchange using amino epoxide supports (8, 9), it will be possible to obtain the same orientation as that described above. Finally, the support would have to be as inert as possible.

Once again, there are various possibilities to obtain these results.

### 2.1. Antibody immobilisation on Eupergit C with 70 micromoles of MANAE groups and 140 micromoles of epoxide groups (support with aminated groups and epoxide groups).

The support was prepared in accordance with the protocol described by Mateo et al. (8). Thus, an epoxide support such as Eupergit C was used, wherein 30%-35% of diamine groups (e.g. ethylenediamine) were introduced, with two amino groups per substituted group. In this manner, a support was obtained with a large number of epoxide groups and, simultaneously, with a relatively large number of amino groups capable of causing antibody adsorption by ionic exchange, the antibody subsequently reacting with the support's epoxide groups.

5 mg of anti-peroxidase antibody in 50 ml of 5 mM phosphate buffer at pH 7 were offered to 5 grams of this Eupergit C amino-epoxide support. After 24 hours, 100% of the antibody had become covalently immobilised on the support, as verified by the non-desorption of the antibody at high ionic strength (although the first step was performed via an adsorption by ionic exchange on the support's amino groups). Subsequently, the epoxide groups were blocked or modified by incubation with 3 M of Asp (it is also possible to use glutamic acid), introducing a net negative charge for each modification, which results in a support that has practically no net charge and, therefore, no capacity to adsorb proteins by ionic exchange at moderate ionic strengths and pH 7 (e.g. 100 mM sodium phosphate).

Subsequently, 1 g of the immobilised antibody was incubated with 1 g of proteins of a crude extract of *E. coli* in 100 ml of 100 mM of sodium phosphate at pH 7, and no adsorption of proteins was detected after 3 hours of incubation. When peroxidase was added to this crude extract, this protein was very quickly adsorbed onto the immobilised antibody preparation. The immobilised antibody functionality was over 80% of that expected on the basis of the immobilised antibody molecules.

### 2.2. Antibody immobilisation on 4BCL agarose activated with 60 micromoles of amino-epoxide groups (epoxide support on a space arm containing an amino group).

This strategy uses supports with epoxide groups the space arm whereof contains an amino group that is capable of acting as an ionic exchanger with the antibody; i.e. wherein, for each epoxide group, there is at least one amino group (if a diamine bound by a secondary amine is used, there could be two amines). These supports will be capable of adsorbing the antibody even more rapidly than in the preceding case, and, furthermore, the covalent bond with the epoxide group could be achieved even faster than in the preceding case. The support was produced by reaction of an aminated support, such as those obtained by the modification of the carboxyl groups of carboxymethyl agarose with ethylenediamine in the presence of 100 mM of carbodiimide, with a diepoxide (e.g. 1,4-butanediol-diglycidyl ether) or other bifunctional epoxide reagents with a group that is more reactive than the epoxide itself (e.g. epichlorhydrin) (13). Although they may produce good results, other groups that introduce two amino groups per epoxide will require a more careful blockage or using a higher pH, in order to cancel one of the charges of the secondary amines.

This made it possible to obtain a support with a dense layer of aminated groups with an epoxide group, as described in Mateo et al. (13).

5 mg of anti-peroxidase antibody in 50 ml of 5 mM phosphate buffer at pH 7 were offered to 5 g of this support. After 24 hours, 100% of the antibody had become covalently immobilised on the support (although the first step was performed via an adsorption by ionic exchange on the support's amino groups, which took place in only 1 hour). Subsequently, following the covalent immobilisation of the antibody, the epoxide groups were blocked by incubation with 3 M of Asp (it is also possible to use glutamic acid), introducing a net negative charge for each modification, which eliminated the support's capacity as an anion exchanger. In those cases where the support has two amino groups, other compounds that introduce two net negative charges will be used. Thus, once again the antibodies will be covalently immobilised with the orientation obtained by anion exchange at pH 7, with a final support surface that is incapable of adsorbing proteins by ionic exchange.

Subsequently, 1 g of the immobilised antibody was incubated with 1 g of proteins of a crude extract of *E. coli* in 100 ml of 100 mM of sodium phosphate at pH 7, and no adsorption of proteins was detected after 3 hours of incubation. When peroxidase was added to this crude extract, this protein was very quickly adsorbed onto the immobilised antibody preparation. The immobilised antibody functionality was over 80% of that expected on the basis of the immobilised antibody molecules.

### REFERENCES

1. Ahluwalia A., DeRossi D., Schirone A., Serra C. 1991. A comparative study of protein immobilization techniques for optical immunosensors. Biosens. Bioelectron. 7: 207-214.
2. Anderson G.P., Jacoby M.A., Ligler F.S., King K.D. 1997. Effectiveness of protein A for antibody immobilization for a fiber optic biosensor. Biosens. Bioelectron. 12: 39-336.
3. Babacam S., Pivarnik P., Lecther S., Rand A.G. 2000. Evaluation of antibody immobilization methods for piezoelectric biosensor application. Biosens. Bioelectron. 15: 615-621.
4. Danczyk R., Krieder B., North T., Webster T., Hogenesch H., Rundell A. 2003. Comparison of antibody functionality using different immobilization methods. Biotechnol. Bioeng. 84: 215-223.
5. Fuentes, Manuel, César Mateo, J.M. Guisán and Roberto Fernández-Lafuente. 2005. Preparation of inert magnetic nano-particles for the directed immobilization of antibodies. Biosens. Bioelec. 20: 1380-1387.
6. Fuentes García, Manuel, César Mateo González, Roberto Fernández Lafuente, José Manuel Guisán Seijas, Carmen Force Redondo, Ignacio Rodríguez Galván, Pedro Tartaj Salvador, Carlos Serna Pereda. "Inmovilización de anticuerpos en partículas magnéticas para detección de trazas de proteínas en fluidos biológicos". Spanish Patent 200202751.
7. Lu B., Smy M.R., O'Kennedy R. 1996. Oriented immobilization of antibodies and its application in immunoassays and immunosensors. Analyst 121: 29R-32R.
8. Mateo C., Fernández-Lorente G., Abian O., Fernández-Lafuente R., Guisán J.M. 2000. Multifunctional epoxy-supports. A new tool to improve the covalent immobilization of proteins: the promotion of physical adsorption of proteins on the supports before their covalent linkage. Biomacromolecules 1: 739-745.
9. Mateo C., Torres R., Fernández G., Ortiz C., Fuentes M., Hidalgo A., López-Gallego F., Betancor L., Pessela B.C.C., Aminati A., Fernández-Lafuente R., Guisán J.M. 2003. Epoxy-amino sepabeads: a new support for immobilization of proteins under mild conditions. Biomacromolecules 4: 772-777.
10. Weetall H.H., Lee M.J. 1989. Antibodies immobilized on inorganic supports. Appl. Biochem. Biotech. 22: 311-330.
11. Zull J.E., Reed-Mundell J., Lee Y.W., Vezenov D., Ziats N.P., Anderson J.M., Sukenik C.N. 1994. Problems and approaches in covalent attachment of peptides and proteins to inorganic surfaces for biosensors applications. J. Indust. Microbiol. 13: 137-143.
12. Lorena Betancor, Fernando López-Gallego, Aurelio Hidalgo, Noelia Alonso-Morales, Gisela Dellamora-Ortiz, César Mateo, Roberto Fernández-Lafuente and José M. Guisán. 2006. Different mechanisms of protein immobilization on glutaraldehyde activated supports: Effect of support activation and immobilization conditions. Enzyme Microb. Technol. 39: 877-882.
13. Benevides C.C., Pessela, Lorena Betancor, Roberto Munilla, Manuel Fuentes, Alfonso V. Carrascosa, Alejandro Vian, Roberto Fernández-Lafuente and José M. Guisán. 2004. Ionic exchange using lowly activated supports: an easy way for purifying large proteins. J. Chromat. A. 1034: 155-159.

## Claims

1. Method for the oriented covalent immobilisation of antibodies, **characterised in that** it comprises the following steps:
i) antibody adsorption by anion exchange on a bifunctional solid surface or support activated with two types of groups: ionised amino groups from the support, composed of groups capable of acting as anion exchangers to define the site of interaction between the antibody and the support, and groups capable of covalently reacting with and immobilising the antibody at low ionic strength,
ii) covalent binding of the already-adsorbed antibody and the support, the conditions whereof will depend on the activated group, and
iii) inertisation or blockage of the support surface.

2. Method, as claimed in claim 1, **characterised in that** the support is activated with two types of groups: groups with a positive charge, preferably amino groups, and groups that are capable of covalently reacting with the proteins, preferably pertaining to the epoxide, aldehyde or glutaraldehyde groups.

3. Method, as claimed in claim 2, **characterised in that** the bifunctional support with positively charged groups is a support that is activated with a low density of glutaraldehyde-activated primary amino groups, capable of adsorbing the antibody by anion exchange at very low ionic strength and, subsequently, covalently immobilising the antibody, with the result that this support is not capable of adsorbing proteins at moderate ionic strength.

4. Method, as claimed in claim 2, **characterised in that** the bifunctional support with positively charged groups is a support that is activated with a low density of epoxide groups on a space arm with an amino group; the amino groups are capable of adsorbing the antibody by anion exchange at very low ionic strength but incapable of adsorbing other proteins at moderate ionic strength, and, subsequently, the epoxide groups are capable of covalently immobilising the antibody and the remaining epoxide groups are blocked or made inert with different molecules that produce a neutral or negative final charge, such that the final charge balance is close to 0.

5. Method, as claimed in claim 4, **characterised in that** the blocking molecules used to produce a neutral final charge belong to the following groups: mercaptoethanol, Gly and Cys.

6. Method, as claimed in claim 4, **characterised in that** the blocking molecules used to produce a negative final charge belong to the following groups: Asp and Glu.

7. Method, as claimed in claim 2, **characterised in that** the bifunctional support with positively charged groups is a support that is activated with a high density of glutaraldehyde groups on amino groups; the amino groups will be capable of adsorbing the antibody by anion exchange at very low ionic strength and, subsequently, the glutaraldehyde groups are capable of covalently immobilising the antibody; in order to eliminate the support's adsorption capacity, it is coated with a layer of small molecular size proteins.

8. Method, as claimed in claim 7, **characterised in that** the space arm of the support with glutaraldehyde-activated amino groups is an amino acid ester or similar; the amino groups will be capable of adsorbing the antibody by anion exchange at very low ionic strength and, subsequently, the glutaraldehyde groups will be capable of covalently immobilising the antibody; in order to eliminate the support's adsorption capacity, it is sufficient to hydrolise the ester to generate a carboxyl group.

9. Method, as claimed in claim 7, **characterised in that** the ester belongs to the following groups: cysteine methyl ester, which is made to react with an epoxide support, an ester of an amino acid such as Lys, which is made to react on a support activated with carboxyl groups, or an ester of an amino acid such as Asp or Glu, which is made to react on a support activated with amino groups.

10. Method, as claimed in claim 2, **characterised in that** the support is activated with a high density of epoxide groups with a space arm that contains at least one positively charged group; this positive charge is capable of promoting antibody adsorption by anion exchange at very low ionic strength and, subsequently, immobilising the antibody by covalent reaction; in order to eliminate the support's adsorption capacity, it is coated with a layer of small molecular size proteins or the epoxide groups are blocked.

11. Method, as claimed in claim 10, **characterised in that** blockage of the epoxide groups is performed using a molecule with a net negative charge pertaining to the following groups: Asp and Glu.

12. Method, as claimed in claim 2, **characterised in that** the support is activated with epoxide groups, ideally one-third whereof have been modified with diamines, preferably ethylenediamine, with two cationic groups; these amino groups are capable of adsorbing the antibody by anion exchange at very low ionic strength and, subsequently, the remaining epoxide groups are capable of covalently immobilising the antibody; in order to eliminate the support's adsorption capacity, it is coated with a layer of proteins of small molecular size or the epoxide groups are blocked with molecules that have a net negative charge (e.g. Asp and Glu).

13. Method, as claimed in claims 1 to 12, **characterised in that** the support is porous.

14. Method, as claimed in claim 10, **characterised in that** the porous support belongs to the following groups: agarose, glass, silica and epoxy-acrylic.

15. Method, as claimed in claims 1 to 12, **characterised in that** the support is a non-porous magnetic nanoparticle.

16. Method, as claimed in claims 1 to 12, **characterised in that** the immobilised antibody is used in affinity chromatography or as an immunosensor.

17. Method, as claimed in claims 1 to 12, **characterised in that** the support is used as an immunosensor or in affinity chromatography in mixtures with particles in suspension; for example, membranes, spores or cells.

18. Use of the method, as claimed in claims 1 to 17, to produce a support device for oriented immobilised antibodies, wherein the support is inert.

19. Inert support device for oriented immobilised antibodies prepared using the method of the invention.
